# EUROPEAN PATENT SPECIFICATION

(11) **EP 0 670 675 B1**
(45) Date of publication and mention of the grant of the patent: **24.03.1999**
(21) Application number: 94901103.5
(22) Date of filing: 11.11.1993
(51) Int. Cl.: A01N 33/08, C10M 133/08

(54) **AN AQUEOUS ALKALINE METAL WORKING FLUID CONTAINING A PRIMARY AMINE**
WÄSSRIGE ALKALIMETALL-BEARBEITUNGSFLÜSSIGKEIT, DIE EIN PRIMÄRES AMIN ENTHÄLT
FLUIDE AQUEUX ALCALIN POUR L'USINAGE DE METAUX, CONTENANT UNE AMINE PRIMAIRE

(30) Priority: 25.11.1992 SE 9203539
(43) Date of publication of application: 13.09.1995
(73) Proprietor: Berol Nobel AB, 444 85 Stenungsund 1 (SE); CASTROL LIMITED, Swindon, Wiltshire SN3 1RE (GB)
(72) Inventor: SKÖLD, Rolf, S-444 41 Stenungsund (SE); RUANE, Patrick, J., Newbury, Berkshire (GB)
(74) Representative: Lewis, Pauline Therese
(86) International application number: SE9300958
(87) International publication number: WO9412028

(56) References cited:
- EP-A- 0 480 841
- WO-A-89/09254
- US-A- 4 196 217
- US-A- 4 589 992
- US-A- 4 749 503
- US-A-47 740 331

## Description

The present invention relates to a water-based metal working fluid containing a primary amine as a corrosion inhibiting and antimicrobial agent. The effects of the primary amine are especially favourable at a pH value above 8.

Water-based alkaline metal working fluids, undergo after some time of use or storage undesirable changes which can be related to the fact that the components included in the fluids are degraded by bacteria, fungi and other microorganisms. The microbial degradation considerably reduces the life and the performance of the fluids. For example the microbial degradation of the fluids may destroy the corrosion inhibiting and lubricating properties. From an economic point of view, it is therefore of great importance to minimize the microbial degradation.

Well-known antimicrobial agents used in metal working fluids are formaldehyde or compounds giving off formaldehyde. Since formaldehyde readily evaporates from the fluid in open systems, the formaldehyde content will be successively reduced and the surrounding air contaminated with formaldehyde at the same time.

Other antimicrobial agents are quaternary ammonium compounds, but their use in metal working fluids, has involved many practical problems, for instance because of reaction with organic acids and anionic surfactants that may be present in the formulation.

The use, as antimicrobial agents, of reaction products of boric acid and a number of organic compounds, such as alkanolamines and carboxylic acids is also known in cutting fluids. However, such reaction products have been found to have a relatively low antimicrobial effect, primarily on fungi, and must therefore be used in relatively large amounts.

From articles by E.O. Bennett, e.g. his article in J.A. Soc. Lubr. Eng., 35 (1979), 137-144, U.S. Patent No.4,749,503, and European Patent Application 412 089, it is known that secondary and tertiary alkanolamine compounds substituted by hydrocarbon groups having 1-18 carbon atoms have an antimicrobial effect in cutting fluids and coolants.

The use of a number of alkoxylated amines and alkanolamines in order to obtain a tool life extension is known from European Patent Applications Nos. 196,810 and 192,358. For metal working fluids containing N-methylethanolamine a lowered susceptibility to the growth of mold and bacteria is reported. From the European Patent 180,561 it is also known that certain tertiary alkanolamines have corrosion inhibiting effects in metal working fluids.

The use of primary alkylamine as an antimicrobial agent in metal working fluids has been suggested. However, their practical application has been hampered because of their inherently irritating and odorous nature combined with relatively high volatility.

S. Watanabe et al disclose in JAOCS, Vol. 68, No. 1 (January 1990), p. 44-46, the use of 3-aminopropyl alkyl ethers in water-based cutting fluids. However, from table 3 in the article it is evident that the 3-aminopropyl alkyl ethers tested could not prevent the bacterial growth in the spent coolant tested.

US Patent No 4 196 217 discloses compounds of the formula

R-X-CH₂-CH(OH)-CH₂NH₂

in which R is a saturated or unsaturated straight-chain C₅ to C₁₈ aliphatic radical and X represent -O-, -S-, -NH- or -CH₂-, the salts, esters and quaternary alkyl ammonium salts of these compounds. The compounds may be used as sterilizing agents and preservatives, especially in the cosmetics industry, and as antiseptics for example in dermatology. US Patent No 4 589 992 discloses the use of salts of primary amines in non-aqueous functional fluids. The salts have the formula

Y-NH₃⁺.RCO₂⁻

in which Y is a group of formula R¹XCH₂CH(OH)CH₂-, R and R¹ are the same or different and each is a straight- or branched chain alkyl group having from 1 to 18 carbon atoms, a straight- or branched chain alkenyl group having from 2 to 18 carbon atoms, a cycloalkyl group having from 4 to 12 ring carbon atoms, an aryl group having 6 to 10 ring carbon atoms or an aralkyl group having from 7 to 11 carbon atoms; and X is O, CO₂, NR² or S and R² is hydrogen, a straight- or branched chain alkyl group having from 1 to 18 carbon atoms, or an alkynyl group having from 2-18 carbon atoms.

Alkanolamines, such as monoethanolamine, diethanolamine and triethanolamine, have frequently been used as corrosion inhibiting agents in aqueous alkaline industrial fluids. From US patents 3 280 029 and 4 976 919, it is well-known to use secondary and tertiary alkyl alkanolamines as corrosion inhibiting agents.

According to the present invention it has now been found that a water-based alkaline metal working fluid containing a lubricant and/or a corrosion inhibitor and having a pH value of at least 8, characterized in that it also contains a primary amine having the formula

R(OCH₂)ₙCH(OH)CH₂NH₂ (I)

in which R is an acyclic hydrocarbon group with 2-12 carbon atoms, n is 0 or 1, or a salt thereof, has low corrosion and microbial degradation. It is a well-known fact that water-based metal working fluids are strongly exposed to bacteria and fungi. However, the primary amines of formula I have proved to be very effective as anticorrosion and antimicrobial agents under the conditions present in water-based synthetic and semi-synthetic metal working fluid. The fact that the primary amines have antimicrobial efficacy also directly supports the maintainance of corrosion inhibiting properties of the same amines. The water-based alkaline fluid of the invention may be in the form of an emulsion, microemulsion, colloidal solution or a true solution.

The primary amines of the formula I may easily be prepared by conventional methods, e.g. by reacting an α-olefin epoxide or an alkyl glycosidyl ether with ammonia. If desired, the primary amine may be used in form of a salt soluble in water and/or oil. Especially preferred are salts with pharmaceutically acceptable anions. Specific examples of salts are phosphates, sulphates, phosphonates, sulphonates and carboxylates. The acyclic hydrocarbon group R in the primary amine of formula (I) may be straight or branched, saturated or unsaturated. Preferably it is a saturated, straight hydrocarbon group with 4-10 carbon atoms. Most preferably R contains 6-8 carbon atoms. Examples of suitable groups are butyl, hexyl, octyl and decyl. Primary amines, where n is 0, have proved to have good antimicrobial effects in emulsions which are normally subject to severe attacks by microorganisms. Primary amines, where n is 1, are preferably used in aqueous solutions. They are easy to incorporate in such systems probably because they exhibit both an ether link and a hydroxyl group. The amount to be added of the primary amines varies from one case to another, but are generally from 0.001-10% by weight, preferably from 0.01-2% by weight.

The metal working fluid according to the invention has preferably a pH value of at least 8, most preferably between 8 and 10, and contains a primary amine having the formula I or a salt thereof in an amount of 0.001 to 10% by weight, preferably 0.01 to 2% by weight.

In addition to the primary amine the metal working fluid contains a lubricant and/or another corrosion inhibitor.

The corrosion inhibitors are normally present in an amount of 0.1-10%, preferably 0.2-3%, by weight of the metal working fluid. Examples of suitable corrosion inhibitors are besides the primary amines of formula I, other amine compounds, such as mono-, di- or triethanolamine, alkali metal hydroxides, triazole or thiadiazole compounds, monocarboxylic acids having 6-11 carbon atoms such as heptanoic acid or isononanoic acid, dicarboxylic acids preferably having 6-12 carbon atoms such as azelaic acid or sebacic acid, alkyl- or aryl-sulphonamidocarboxylic acids; inorganic acids, such as boric acid; and conventional reaction products between boric acid and/or carboxylic acids with organic compounds, such as alkanolamines. Examples of corrosion inhibitors are also the amine compounds described in European Publication No. 180,561.

In order to increase the friction-reducing capacity, the metal working fluids may also contain lubricants. They are usually selected from the group consisting of oils; esters or amides of mono- or dicarboxylic acids having at least 10 carbon atoms in the acyl groups; monocarboxylic acids having 12 or more carbon atoms; dicarboxylic acid having more than 12 carbon atoms; organic phosphate esters containing one or two hydrocarbon groups having 6-18 carbon atoms; nonionic alkylene oxide adducts having a molecular weight above 400, such as polypropylene glycol or randomly distributed polypropylene-ethylene glycols or block polymers of ethylene and propylene oxide and mixtures thereof; and oils. The amount of the lubricant is 0.05-10%, preferably 0.1-2%, by weight of the metal working fluid. Preferably the monocarboxylic acid lubricants are coconut fatty acids, oleic acid, groundnut acids and rapeseed acids and esters and amides of these acids with polyols, such as glycerol, trimethylolpropane, pentaerythritol and polyalkylene glycols, and alkanolamines respectively. The hydrocarbon groups of the organic phosphate esters can be octyl, nonyl, decyl,dodecyl,tetradecyl and hexadecyl as well as their corresponding unsaturated alkenyl groups. Anionic lubricants also have a corrosion-preventing capacity against iron.

The metal working compounds containing an oil as a lubricant have often the form of an emulsion or a colloidal solution. With the term "oil" is here understood a class of substances of synthetic, mineral, vegetable or animal origin. Usually, they are from petroleum or are petroleum-derived but synthetic hydrocarbons such as poly-alpha-olefins (PRO's) or alkylates such as alkyl benzenes are also used. These compositions also include emulsifying agents which are usually nonionic and/or anionic surfactants. Examples of anionic surfactants are alkylaryl sulphonates, such as dodecylbenzene sulphonates, alkylsulphates; such as sulphates of alcohols or alkoxylated alcohols; sulphated esters, such as sulphated castor oil; and phosphates of alcohols or ethoxylated alcohols. Examples of nonionic surfactants are alkoxylated alkyl phenols, alcohols, carboxylic acids, alkanolamines, alkylamines and alkylamides. The alkoxylation agent is normally an alkylene oxide containing 2-4 carbon atoms. Preferably at least 50% of the alkyleneoxy groups are ethyleneoxy groups and they may be either arranged in blocks or distributed at random. In a preferred embodiment the polyoxyalkylene is end-capped with propyleneoxy and/or butyleneoxy units in order to obtain a low-foaming surfactant. The anionic and nonionic surfactants are normally so chosen that they contain 8-20 carbon atoms in a hydrocarbon residue. By the amount of ethyleneoxy units in the surfactant the HLB-balance can be further regulated.

In addition to corrosion inhibitors and lubricants, the metal working fluid may advantageously also contain pH-adjusting agents, metal complex stabilizers, defoamers, perfumes, viscosity-adjusting and solubility-improving agents in known manner. Suitable solubility-improving agents are glycols, such as hexylene glycol; alcohols, such as tridecanol and oleylalcohol; and glycol ethers, such as butyldioxitol and butyltrioxitol.

The present invention is further illustrated by the following Examples.

### Example 1

A semi-synthetic metal working fluid concentrate having an amine concentration of 5% by weight was prepared from the following components.

| **Components** | **% by weight** |
|---|---|
| Refined paraffinic oil | 40.8-48.3 |
| sodium petroleum sulphonate (av Mwt 440) | 14.0 |
| Oleic acid | 10.0 |
| Chlorinated paraffin (65% chlorine) | 8.0 |
| 50% aqueous potassium hydroxide | 3.2 |
| Water | 2.0 |
| Neopentyl glycol dioleate | 5.0 |
| Hexylene glycol | 4.0-10.0 |
| Tridecanol, branched | 0.5-2.0 |
| Amine in accordance with tables 1-4 | 5.0 |

In order to obtain homogeneous compositions hexylene glycol and tridecanol may be incorporated in larger amounts than the minimum amount in the table above. The increased amounts added are balanced by corresponding reductions in the amount of the refined paraffinic oil.

Anticorrosion performance was established using a modified version of The Institute of Petroleum IP 287/82 test method. In the modified procedure deionised water was used instead of a synthetic sea water of 200 ppm of calcium carbonate in the preparation of the test emulsion. Cast iron chips were placed on Whatman number 6 filter paper and wetted with the above formulation diluted with water for 2 hours at ambient temperature. The percentage area stained was recorded.

The following tables show the anticorrosion performance where emulsions were tested at pH 9.0 and 9.5. pH of the formulations was varied using acetic acid/KOH.

**Table 1**

| Corrosion tests using Alkyl-CH(OH)CH₂NH₂ at pH 9.0 | | | | | | |
|---|---|---|---|---|---|---|
| Dilution | Percent corrosion area for alkyl | | | | | |
| | - | C₄H₉ | C₆H₁₃ | C₈H₁₇ | C₁₀H₂₁ | AMP 100¹⁾ |
| 5:1 | 1 | 0 | 0 | 0 | 0 | 10 |
| 10:1 | 2 | 2 | 2 | 0 | 0 | 10 |
| 15:1 | 2 | 2 | 2 | 3 | 1 | 10 |

| | | | | | | |
|---|---|---|---|---|---|---|
| ¹⁾ corrosion inhibitor (aminomethyl propanol from Angus Chemical Co) | | | | | | |

**Table 2**

| Corrosion tests using Alkyl-CH(OH)CH₂NH₂ at pH 9.5 | | | | | | |
|---|---|---|---|---|---|---|
| Dilution | Percent corrosion area for alkyl | | | | | |
| | - | C₄H₉ | C₆H₁₃ | C₈H₁₇ | C₁₀H₂₁ | AMP 100¹⁾ |
| 5:1 | 1 | 0 | 0 | 0 | 0 | 5 |
| 10:1 | 2 | 0 | 0 | 0 | 0 | 6 |
| 15:1 | 2 | 0 | 2 | 1 | 1 | 18 |

| | | | | | | |
|---|---|---|---|---|---|---|
| ¹⁾ corrosion inhibitor (aminomethyl propanol from Angus Chemical Co) | | | | | | |

**Table 3**

| Corrosion tests using Alkyl-0CH₂CH(OH)CH₂ NH₂ at pH 9.0 | | | | | | |
|---|---|---|---|---|---|---|
| Dilution | Percent corrosion area for alkyl | | | | | |
| | - | C₄H₉ | C₆H₁₃ | C₈H₁₇ | C₁₀H₂₁ | AMP 100¹⁾ |
| 5:1 | 1 | 0 | 0 | 0 | 1 | 10 |
| 10:1 | 2 | 0 | 0 | 0 | 1 | 10 |
| 15:1 | 2 | 0 | 0 | 1 | 1 | 10 |

| | | | | | | |
|---|---|---|---|---|---|---|
| ¹⁾ corrosion inhibitor (aminomethyl propanol from Angus Chemical Co) | | | | | | |

**Table 4**

| Corrosion tests using Alkyl-0(CH₂)CH(OH)CH₂NH₂ at pH 9.5 | | | | | | |
|---|---|---|---|---|---|---|
| Dilution | Percent corrosion area for alkyl | | | | | |
| | - | C₄H₉ | C₆H₁₃ | C₈H₁₇ | C₁₀H₂₁ | AMP 100¹⁾ |
| 5:1 | 1 | 0 | 0 | 0 | 1 | 5 |
| 10:1 | 2 | 0 | 0 | 0 | 1 | 6 |
| 15:1 | 2 | 0 | 0 | 0 | 1.5 | 18 |

| | | | | | | |
|---|---|---|---|---|---|---|
| ¹⁾ corrosion inhibitor (aminomethyl propanol from Angus Chemical Co) | | | | | | |

The corrosion tests show that the primary amines exhibit a significant corrosion reducing effect at moderate alkaline pH values.

### Example 2

Semi-synthetic and synthetic metalworking fluids were prepared from the following base formulations with the amines disclosed in tables 5-8.

| **Semi-synthetic concentrate formulation synthetic concentrate formulation** | | | |
|---|---|---|---|
| | % | | % |
| Refined paraffinic oil | 14.2-38.2 | Triethanolamine | 47.4 |
| Anionic surfactant | 7.0 | KOH (50%) | 6.4 |
| Naphthenic acid | 5.5 | Sebacic acid | 16.5 |
| Non-ionic surfactant | 10.0 | Water | 9.7-25.7 |
| Synthetic Hydrocarbon | 17.0 | Amine | 4.0-20.0 |
| Tall oil amide | 8.0 | | |
| Water | 6.0 | | |
| KOH (50%) | 2.3 | | |
| Tridecanol, branched | 2.0-20.0 | | |
| Amine in accordance | | | |
| with table 1-4 | 4.0-20 | | |

The amines were added in amounts of 4, 10 and 20% to provide 1000, 2500 and 5000 ppm amine when diluted with water to a fluid concentration of 2.5%. In order to obtain homogeneous compositions tridecanol may be added in larger amounts than the minimum amounts in the table above. The increased amounts added are balanced by corresponding reductions in the amount of the mineral oil. The diluted fluids were subsequently tested with respect to their bactericidal and fungicidal effects by adding standardised bacterial and fungal inocula which were originally isolated from contaminated metalworking fluids. The inocula used in the tests were prepared as described below.

### Standardised Inoculum Preparation

### (i) Bacteria

100 ml mineral salts media containing 2% trisodium citrate as sole carbon source (pH 9.0) in Erlenmyer flasks were inoculated with 1 ml of a culture of Pseudomonas aeruginosa at an optical cell density of 2.0 measured at 650 nm (Perkin-Elmer UV-Vis Spectrophotometer, model Lambda 2). These inocula were incubated at 30°C in an orbital incubator rotating at 200 rpm. During exponential growth (18 hour culture), further media were sub-inoculated in the same way, and the remaining culture harvested for testing of the diluted formulations. This subculturing procedure was continued until the end of the test period.

The cultures were harvested by centrifugation at 4000 rpm for 20 minutes (MSE Mistral 2000). The resulting bacterial pellet was resuspended in sterile Hanks saline buffer solution and recentrifuged. Three such washes were performed. Prior to the final wash, the optical density was adjusted to 2.0 (650 nm) and the volume of suspension noted. After the third wash the bacterial cells were resuspended in Hanks saline buffer to one tenth of the original volume to provide a concentrated inoculum containing approximately 1 x 10¹⁰ cells ml⁻¹.

This suspension was used as inoculum in the tests.

### (ii) Fungi

100 ml mineral salts media containing 2% glucose as sole carbon source were introduced into Erhlenmyer flasks and inoculated with 1 ml of a homogenised culture of Cephalosporium sp. The inocula were incubated at 30°C in an orbital incubator rotating at 200 rpm. After 24 hours the fungus was homogenised and subcultured as already described into glucose supplemented mineral salts media. The remaining culture was centrifuged at 4000 rpm for 20 minutes. After decanting the spent growth medium, the fungal pellet was resuspended in Hanks saline buffer and recentrifuged. After 3 washes, the final fungal pellet was resuspended in one tenth of the original volume of buffer used to provide a concentrated inoculum. This material was used as inoculum in the testing of the diluted formulations.

### Test method

2.5 ml of the formulations were diluted with 97.5 ml of sterile mineral salts media introduced in 250 ml Erlenmyer flasks. These dilutions were adjusted to pH 9.5 by adding HCl or KOH. 200 microlitres of the standardised inocula were then added, either daily for the full experimental period providing a multiple inoculation, or singly, at the start of the test with an inoculum consisting of a cell density equivalent to the cumulative multiple inoculum. In this way, the investigation compared (i) the efficacy of the said amines following repeated additions where fresh supplementary biomass was introduced over a period to simulate a continuous contamination situation (i.e multiple inoculum test) and (ii) the efficacy of the said amines where a single presentation of biomass is performed and the long term effects on growth or survival can be monitored independently from the addition of fresh biomass (i.e single inoculation test). Total biomass levels in both tests were comparable.

Both fluid types were inoculated separately with the bacterial and fungal biomass to avoid possible inhibitory interactions. The semi-synthetic fluids were tested over an experimental period of 28 days, whereas satisfactory differentiation of amine performance could be achieved after 14 days in the synthetic fluids. All fluids were incubated throughout the test at 30°C in an orbital incubator rotating at 200 rpm.

The survival of inocula, both in the multiple and single tests was monitored daily. Fungi were monitored using conventional plate counting following growth on malt extract agar (plus chloramphenicol) after serial dilution. Bacteria were enumerated directly using the rapid automated bacterial impedance technique (RABIT).

The following results were obtained.

**Table 5**

| Alkyl-CH(OH)CH₂ NH₂. Efficacy against Acremonium sp. | | | | | | |
|---|---|---|---|---|---|---|
| Alkyl | Inoculation | Formulation | Amine, ppm | | | |
| | | | 0 | 1000 | 2500 | 5000 |
| | | | Mean log₁₀ cfu/ml | | | |
| C₄H₉ | Single | semi-synth | 3.28 | 0 | 0 | 0 |
| C₆H₁₃ | Single | semi-synth | 4.88 | 0 | 0 | 0 |
| C₈H₁₇ | Single | semi-synth | 4.88 | 1.31 | 0.97 | 0 |
| C₁₀H₂₁ | Single | semi-synth | 4.88 | 1.19 | 1.07 | 0.90 |
| C₄H₉ | multiple | semi-synth | 3.85 | 3.84 | 3.52 | 2.17 |
| C₆H₁₃ | multiple | semi-synth | 5.17 | 4.18 | 3.10 | 0 |
| C₈H₁₇ | multiple | semi-synth | 5.17 | 3.11 | 1.31 | 0 |
| C₁₀H₂₁ | multiple | semi-synth | 5.17 | 3.55 | 1.13 | 0.58 |
| C₄H₉ | single | synthetic | 5.56 | 0 | 0 | 0 |
| C₆H₁₃ | single | synthetic | 5.56 | 0 | 0 | 0 |
| C₈H₁₇ | single | synthetic | 5.56 | 0 | 0 | 0 |
| C₁₀H₂₁ | single | synthetic | 5.56 | 0 | 0 | 0 |
| C₄H₉ | multiple | synthetic | 2.87 | 2.87 | 1.88 | 0.71 |
| C₆H₁₃ | multiple | synthetic | 5.54 | 1.27 | 1.58 | 0.34 |
| C₈H₁₇ | multiple | synthetic | 2.87 | 1.16 | 0.39 | 0 |
| C₁₀H₂₁ | multiple | synthetic | 2.87 | 0.87 | 0.37 | 0 |

**Table 6**

| Alkyl-CH(OH)CH₂NH₂. Efficacy against Pseudonomas aeruginosa. | | | | | | |
|---|---|---|---|---|---|---|
| Alkyl chain | Inoculation | Formulation | Amine, ppm | | | |
| | | | 0 | 1000 | 2500 | 5000 |
| | | | Mean log₁₀ cfu/ml | | | |
| C₄H₉ | Single | semi-synth | 8.29 | 7.84 | 7.31 | 0 |
| C₆H₁₃ | Single | semi-synth | 9.30 | 7.45 | 0 | 0 |
| C₈H₁₇ | Single | semi-synth | 9.30 | 7.23 | 2.65 | 0 |
| C₁₀H₂₁ | Single | semi-synth | 9.30 | 8.19 | 7.96 | 7.85 |
| C₄H₉ | multiple | semi-synth | 8.71 | 7.56 | 7.59 | 3.18 |
| C₆H₁₃ | multiple | semi-synth | 9.31 | 6.59 | 1.82 | 0 |
| C₈H₁₇ | multiple | semi-synth | 9.31 | 7.07 | 6.16 | 0 |
| C₁₀H₂₁ | multiple | semi-synth | 9.31 | 7.72 | 7.91 | 6.97 |
| C₄H₉ | single | synthetic | 8.63 | 6.32 | 0 | 0 |
| C₆H₁₃ | single | synthetic | 7.09 | 0 | 0 | 0 |
| C₈H₁₇ | single | synthetic | 8.63 | 0 | 0 | 0 |
| C₁₀H₂₁ | single | synthetic | 8.63 | 7.49 | 7.47 | 7.32 |
| C₄H₉ | multiple | synthetic | 8.46 | 7.75 | 5.09 | 0.59 |
| C₆H₁₃ | multiple | synthetic | 6.87 | 1.82 | 0 | 0 |
| C₈H₁₇ | multiple | synthetic | 8.46 | 0 | 0 | 0 |
| C₁₀H₂₁ | multiple | synthetic | 8.46 | 7.97 | 7.37 | 6.39 |

**Table 7**

| Alkyl-OCH₂CH(OH)CH₂NH₂. Efficacy against Acremonium sp. | | | | | | |
|---|---|---|---|---|---|---|
| Alkyl chain | Inoculation | Formulation | Amine, ppm | | | |
| | | | 0 | 1000 | 2500 | 5000 |
| | | | Mean log₁₀ cfu/ml | | | |
| C₄H₉ | Single | semi-synth | 3.87 | 3.12 | 0.89 | 0.31 |
| C₆H₁₃ | Single | semi-synth | 3.87 | 0.39 | 0.38 | 0 |
| C₈H₁₇ | Single | semi-synth | 3.87 | 1.22 | 0.36 | 0 |
| C₁₀H₂₁ | Single | semi-synth | 3.87 | 3.16 | 0.41 | 0.18 |
| C₄H₉ | multiple | semi-synth | 4.29 | 4.54 | 4.32 | 3.60 |
| C₆H₁₃ | multiple | semi-synth | 4.29 | 3.30 | 3.77 | 1.10 |
| C₈H₁₇ | multiple | semi-synth | 4.29 | 4.36 | 3.36 | 0.12 |
| C₁₀H₂₁ | multiple | semi-synth | 4.29 | 4.49 | 3.67 | 0.44 |
| C₄H₉ | single | synthetic | 3.21 | 1.20 | 0.85 | 0.37 |
| C₆H₁₃ | single | synthetic | 3.21 | 0.10 | 0 | 0 |
| C₈H₁₇ | single | synthetic | 3.21 | 0 | 0.10 | 0 |
| C₁₀H₂₁ | single | synthetic | 3.21 | 0 | 0 | 0 |
| C₄H₉ | multiple | synthetic | 4.67 | 4.35 | 3.77 | 1.67 |
| C₆H₁₃ | multiple | synthetic | 4.67 | 2.35 | 0.10 | 0 |
| C₈H₁₇ | multiple | synthetic | 4.67 | 0 | 0 | 0 |
| C₁₀H₂₁ | multiple | synthetic | 4.67 | 0 | 0 | 0 |

**Table 8**

| Alkyl-OCH₂CH(OH)CH₂NH₂. Efficacy against Pseudomonas aeruginosa. | | | | | | |
|---|---|---|---|---|---|---|
| Alkyl chain | Inoculation | Formulation | Amine, ppm | | | |
| | | | 0 | 1000 | 2500 | 5000 |
| | | | Mean log₁₀ cfu/ml | | | |
| C₄H₉ | Single | semi-synth | 8.55 | 7.87 | 7.22 | 7.43 |
| C₆H₁₃ | Single | semi-synth | 8.55 | 7.76 | 2.60 | 0.10 |
| C₈H₁₇ | Single | semi-synth | 8.55 | 7.93 | 4.66 | 0 |
| C₁₀H₂₁ | Single | semi-synth | 8.55 | 7.87 | 7.65 | 8.20 |
| C₄H₉ | multiple | semi-synth | 9.69 | 7.46 | 8.22 | 7.37 |
| C₆H₁₃ | multiple | semi-synth | 9.69 | 7.37 | 4.36 | 0.91 |
| C₈H₁₇ | multiple | semi-synth | 9.69 | 7.67 | 5.84 | 0.50 |
| C₁₀H₂₁ | multiple | semi-synth | 9.69 | 7.23 | 7.61 | 6.55 |
| C₄H₉ | single | synthetic | 7.75 | 6.35 | 5.16 | 0.93 |
| C₆H₁₃ | single | synthetic | 7.75 | 0 | 0 | 0 |
| C₈H₁₇ | single | synthetic | 7.75 | 0 | 0 | 0 |
| C₁₀H₂₁ | single | synthetic | 7.75 | 0 | 0 | 0 |
| C₄H₉ | multiple | synthetic | 7.53 | 6.03 | 5.40 | 1.71 |
| C₆H₁₃ | multiple | synthetic | 7.53 | 1.92 | 0 | 0 |
| C₈H₁₇ | multiple | synthetic | 7.53 | 0 | 0 | 0 |
| C₁₀H₂₁ | multiple | synthetic | 7.53 | 0 | 0 | 0 |

It is evident that the primary amines have excellent anti-microbial effects. Especially good results are shown by the hydroxyalkyl amines in the semi-synthetic formulations, while the hydroxy ether amines exhibit excellent results in the synthetic formualtions.

## Claims

1. An water-based alkaline metal working fluid containing a lubricant and/or a corrosion inhibitor and having a pH value of at least 8 **characterized** in that it also contains a primary amine R(OCH₂)ₙCH(OH)CH₂NH₂, where R is an acyclic hydrocarbon group with 2-12 carbon atoms and n is 0 or 1, or a salt thereof, in an amount of 0.001 to 10% by weight, preferably 0.01-2% by weight.

2. The metal working fluid in claim 1, where R is an alkyl group with 4-10 carbon atoms.

3. An emulsion of the metalworking fluid in claim 1 or 2 wherein n in the primary amine is 0.

4. A solution of the metal working fluid in claim 1 or 2 wherein n in the primary amine is 1.

5. Use of a primary amine having the formula
R(OCH₂)ₙCH(OH)CH₂NH₂
where R is an acyclic hydrocarbon group with 2-12 carbon atoms and n is 0 or 1, or a salt thereof as a corrosion inhibiting and antimicrobial agent in a water-based alkaline metal working fluid containing a lubricant and/or a corrosion inhibitor.

6. Use in claim 5 where R is an alkyl group with 4-10 carbon atoms.

## Patentansprüche

1. Alkalische Metallbearbeitungsflüssigkeit auf Wasserbasis, die ein Schmiermittel und/oder einen Korrosionsinhibitor enthält sowie einen pH-Wert von mindestens 8 aufweist, dadurch gekennzeichnet, daß sie auch ein primäres Amin R(OCH₂)ₙCH(OH)CH₂NH₂, worin R ein acyclischer Kohlenwasserstoffrest mit 2 bis 12 Kohlenstoffatomen und n die Zahl 0 oder 1 bedeuten, oder ein Salz hiervon in einer Menge von 0,001 bis 10 Gew%, vorzugsweise von 0,01 bis 2 Gew%, enthält.

2. Metallbearbeitungsflüssigkeit nach Anspruch 1, worin R einen Alkylrest mit 4 bis 10 Kohlenstoffatomen bedeutet.

3. Emulsion der Metallbearbeitungsflüssigkeit nach Anspruch 1 oder 2, worin n in dem primären Amin die Zahl 0 bedeutet.

4. Lösung der Metallbearbeitungsflüssigkeit nach Anspruch 1 oder 2, worin n in dem primären Amin die Zahl 1 bedeutet.

5. Verwendung eines primären Amins mit der Formel
R(OCH₂)ₙCH(OH)CH₂NH₂,
worin R einen acyclischen Kohlenwasserstoffrest mit 2 bis 12 Kohlenstoffatomen und n die Zahl 0 oder 1 bedeuten, oder eines Salzes hiervon als korrosionsinhibierendes und antimikrobielles Mittel in einer alkalischen Metallbearbeitungsflüssigkeit auf Wasserbasis, die ein Schmiermittel und/oder einen Korrosionsinhibitor enthält.

6. Verwendung nach Anspruch 5, worin R einen Alkylrest mit 4 bis 10 Kohlenstoffatomen bedeutet.

## Revendications

1. Fluide aqueux alcalin pour l'usinage de métaux contenant un lubrifiant et/ou un inhibiteur de corrosion et ayant une valeur de pH d'au moins 8 caractérisé par le fait qu'il contient également une amine primaire R(OCH₂)ₙCH(OH)CH₂NH_{2,} R étant un groupe hydrocarboné acyclique ayant de 2-12 atomes de carbone et n est 0 ou 1 ou un sel correspondant, en une quantité de 0,001 à 10% en poids, de préférence 0,01-2% en poids.

2. Fluide pour l'usinage de métaux selon la revendication 1 dans lequel R est un groupe alkyle ayant 4-10 atomes de carbone.

3. Emulsion du fluide pour l'usinage de métaux selon la revendication 1 ou 2 dans laquelle n dans l'amine primaire est 0.

4. Solution de fluide pour l'usinage de métaux selon la revendication 1 ou 2 dans laquelle n dans l'amine primaire est 1.

5. Utilisation d'une amine primaire ayant la formule
R(OCH₂)ₙCH(OH)CH₂NH₂
dans laquelle R est un groupe hydrocarboné acyclique ayant 2-12 atomes de carbone et n est O ou 1, ou un sel correspondant, en tant qu'inhibiteur de corrosion et d'agent anti-microbien dans un fluide aqueux alcalin pour l'usinage dans un fluide aqueux alcalin pour l'usinage de métaux contenant un lubrifiant et/ou un inhibiteur de corrosion.

6. Utilisation selon la revendication 5, dans laquelle R est un groupe alkyle ayant 4-10 atomes de carbone.
